# EUROPÄISCHE PATENTSCHRIFT

(11) **EP 1 915 376 B1**
(45) Veröffentlichungstag und Bekanntmachung des Hinweises auf die Patenterteilung: **09.09.2009**
(21) Anmeldenummer: 06777865.4
(22) Anmeldetag: 20.07.2006
(51) Int. Cl.: C07D 487/04

(54) **FUNGIZIDE 6-PHENYL-PYRAZOLOPYRIMIDIN-7-YLAMINE**
6-PHENYL-PYRAZOLOPYRIMIDINE-7-YLAMINE FUNGICIDES
6-PHENYLE-PYRAZOLOPYRIMIDINE-7-YLAMINES FONGICIDES

(30) Priorität: 27.07.2005 DE 102005035686
(43) Veröffentlichungstag der Anmeldung: 30.04.2008
(73) Patentinhaber: BASF SE, 67056 Ludwigshafen (DE)
(72) Erfinder: DIETZ, Jochen, 68167 Mannheim (DE); GRAMMENOS, Wassilios, 67071 Ludwigshafen (DE); HÜNGER, Udo, 68167 Mannheim (DE); LOHMANN, Jan Klaas, 68167 Mannheim (DE); RENNER, Jens, 68163 Mannheim (DE); RHEINHEIMER, Joachim, 67063 Ludwigshafen (DE)
(86) Internationale Anmeldenummer: PCT/EP2006/064468
(87) Internationale Veröffentlichungsnummer: WO 2007/012601

(56) Entgegenhaltungen:
- EP-A- 0 071 792

## Beschreibung

Die vorliegende Erfindung betrifft 6-Phenyl-pyrazolopyrimidin-7-ylamine der Formel I, 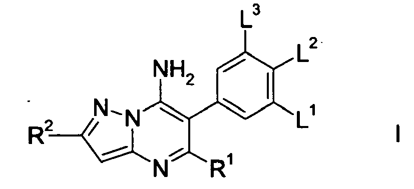 in der die Substituenten folgende Bedeutung haben:
- L¹,L²,L³: unabhängig voneinander Wasserstoff, Halogen, Hydroxy, Mercapto, Nitro, NR^{A}R^{B}, C₁-C₁₀-Alkyl, C₁-C₄-Halogenalkyl, C₂-C₆-Alkenyl, C₂-C₆-Alkinyl, C₁-C₈-Alkoxy, Phenyl, Phenoxy, Phenylthio, Benzyloxy und Benzylthio; R^{A}, R^{B} Wasserstoff und C₁-C₆-Alkyl;
wobei zwei benachbarte Gruppen aus L¹, L² und L³ gemeinsam eine C₁-C₄-Alkylen, C₂-C₄-Oxyalkylen, C₁-C₃-Oxyalkylenoxy oder Butadienylgruppe darstellen können;
wobei mindestens eine Gruppe L¹, L² oder L³ ungleich Wasserstoff ist und die Gruppen L¹, L² oder L³ unsubstituiert oder durch eine bis vier gleiche oder verschiedene Gruppen Ra substituiert sind:
Ra Halogen, Cyano, Hydroxy, Mercapto, C₁-C₁₀-Alkyl, C₁-C₁₀-Halogenalkyl, C₃-C₈-Cycloalkyl, C₂-C₁₀-Alkenyl, C₂-C₁₀-Alkinyl, C₁-C₆-Alkoxy, C₁-C₆-Alkylthio, C₁-C₆-Alkoxy-C₁-C₆-alkyl oder NR^{A}R^{B};
- R¹: C₁-C₄-Halogenalkyl, C₂-C₆-Alkenyl, C₂-C₆-Alkinyl, C₂-C₈-Alkoxyalkyl oder Phenyl-C₁-C₂-alkyl, wobei der Ring unsubstituiert oder durch eine oder mehrere Gruppen Halogen oder C₁-C₈-Alkyl substituiert sein kann;
- R²: Wasserstoff, Halogen, Cyano, NR^{A}R^{B}, Hydroxy, Mercapto, C₁-C₆-Alkyl, C₁-C₆-Halogenalkyl, C₃-C₈-Cycloalkyl, C₁-C₆-Alkoxy, C₁-C₆-Alkylthio, C₃-C₈-Cycloalkoxy, C₃-C₈-Cycloalkylthio, Carboxyl, Formyl, C₁-C₁₀-Alkylcarbonyl, C₁-C₁₀-Alkoxycarbonyl, C₂-C₁₀-Alkenyloxycarbonyl, C₂-C₁₀-Alkinyloxycarbonyl, Phenyl, Phenoxy, Phenylthio, Benzyloxy, Benzylthio, C₁-C₆-Alkyl-S(O)ₘ- und fünf- oder sechsgliedriger gesättigter, partiell ungesättigter oder aromatischer Heterocyclus, enthaltend ein bis vier Heteroatome aus der Gruppe O, N oder S;
m 0, 1 oder 2; wobei die cyclischen Gruppen in L¹, L², L³, R^{a} und/oder R¹ unsubstituiert oder durch eine bis vier Gruppen R^{b} substituiert sind:
R^{b} Halogen, Cyano, Hydroxy, Mercapto, Nitro, NR^{A}R^{B}, C₁-C₁₀-Alkyl, C₁-C₆-Halogenalkyl, C₂-C₆-Alkenyl, C₂-C₆-Alkinyl, C₁-C₆-Alkoxy und fünf- oder sechsgliedriger gesättigter, partiell ungesättigter oder aromatischer Heterocyclus, enthaltend ein bis vier Heteroatome aus der Gruppe O N oder S, welcher unsubstituiert oder durch eine oder mehrere Gruppen Halogen und/oder C₁-C₄-Alkyl substituiert sein kann.

Außerdem betrifft die Erfindung Verfahren zur Herstellung dieser Verbindungen, sie enthaltende Mittel sowie ihre Verwendung zur Bekämpfung von pflanzenpathogenen Schadpilzen.

Aus EP-A 71 792 sind einzelne fungizid wirksame 6-Phenyl-pyrazolopyrimidinylamine bekannt. Ihre Wirkung ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Des weiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus EP-A 71 792 bekannten Verbindungen im wesentlichen durch die Substituion in Position 2 und 5 des Pyrazolopyrimidin-Gerüstes.

Die Verbindungen der Formel I weisen eine gegenüber den bekannten Verbindungen erhöhte Wirksamkeit gegen Schadpilze auf.

Die erfindungsgemäßen Verbindungen können auf verschiedenen Wegen erhalten werden. Vorteilhaft werden die erfindungsgemäßen Verbindungen erhalten, indem man substituierte β-Ketoestern der Formel II mit Aminopyrazolen der Formel III zu 7-Hydroxypyrazolopyrimidinen der Formel IV umsetzt. Die Gruppen L¹ bis L³ und R¹ in Formeln II und IV haben die Bedeutungen wie für Formel I und die Gruppe R in Formel II bedeutet C₁-C₄-Alkyl, aus praktischen Gründen ist Methyl, Ethyl oder Propyl darin bevorzugt.

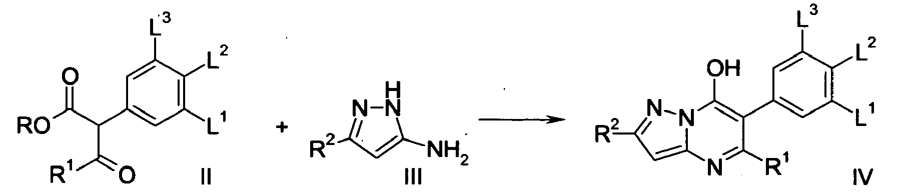

Die Umsetzung der substituierten β-Ketoester der Formel II mit den Aminopyrazolen der Formel III kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe, wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Triisopropylamin, Tributylamin und N-Methylpiperidin, N-Methylmorpholin, Pyridin, substituierte Pyridine wie Collidin, Lutidin und 4-Dimethylaminopyridin sowie bicyclische Amine und Mischungen dieser Lösungsmittel mit Wasser in Frage. Als Katalysatoren kommen Basen, wie voranstehend genannt, oder Säuren, wie Sulfonsäuren oder Mineralsäuren in Frage. Besonders bevorzugt wird die Umsetzung ohne Lösungsmittel oder in Chlorbenzol, Xylol, Dimethylsulfoxid, N-Methylpyrrolidon durchgeführt. Besonders bevorzugte Basen sind tertiäre Amine wie Tri-isopropylamin, Tributylamin, N-Methylmorpholin oder N-Methylpiperidin. Die Temperaturen liegen zwischen 50 und 300°C, vorzugsweise bei 50 bis 180°C, wenn in Lösung gearbeitet wird [vgl. EP-A 770 615; Adv. Het. Chem. Bd. 57, S. 81ff. (1993)].

Die Basen werden im allgemeinen in katalytischen Mengen eingesetzt, sie können aber auch äquimolar, im Überschuss oder gegebenenfalls als Lösungsmittel verwendet werden.

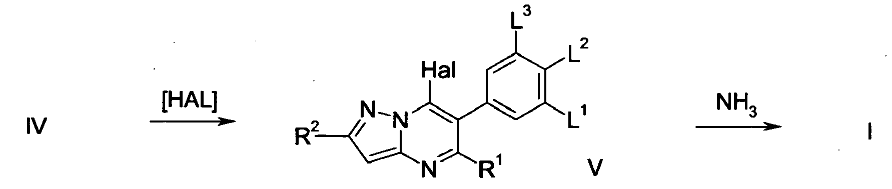

Die so erhaltenen Kondensationsprodukte der Formel IV fallen aus den Reaktionslösungen meist in reiner Form aus und werden nach dem Waschen mit dem gleichen Lösungsmittel oder mit Wasser und anschließendem Trocknen mit Halogenierungsmitteln, insbesondere Chlorierungs- oder Bromierungsmittel zu den Verbindungen der Formel V, in der Hal für Chlor oder Brom, insbesondere für Chlor steht, umgesetzt. Bevorzugt erfolgt die Umsetzung mit Chlorierungsmitteln, wie Phosphoroxychlorid, Thionylchlorid oder Sulfurylchlorid bei 50°C bis 150°C vorzugsweise in überschüssigem Phosphoroxitrichlorid bei Rückflusstemperatur. Nach dem Verdampfen des überschüssigen Phosphoroxitrichlorids wird der Rückstand mit Eiswasser gegebenenfalls unter Zusatz eines mit Wasser nicht mischbaren Lösungsmittels behandelt. Das aus der getrockneten organischen Phase gegebenenfalls nach Verdampfung des inerten Lösungsmittels isolierte Chlorierungsprodukt ist meist sehr rein und wird anschließend mit Ammoniak in inerten Lösungsmitteln bei 100°C bis 200°C zu den 7-Amino-pyrazolopyrimidinen umgesetzt. Die Reaktion wird vorzugsweise mit 1- bis 10-molarem Überschuss an Ammoniak unter Druck von 1 bis 100 bar durchgeführt.

Die neuen Pyrazolopyrimidin-7-ylamine werden gegebenenfalls nach Verdampfen des Lösungsmittels durch Digerieren in Wasser als kristalline Verbindungen isoliert.

Die β-Ketoester der Formel II können hergestellt werden wie in Organic Synthesis Coll. Vol. 1, S. 248 beschrieben, bzw. sind kommerziell erhältlich.

Alternativ können die neuen Verbindungen der Formel I erhalten werden, indem man substituierte Acylcyanide der Formel VI, in der L¹ bis L³ die oben angegebenen Bedeutungen haben, mit Aminopyrazolen der Formel III umsetzt.

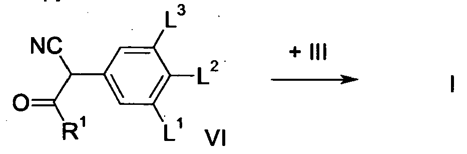

Die Umsetzung kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe, wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie voranstehend genannt, und Mischungen dieser Lösungsmittel mit Wasser in Frage. Die Umsetzungstemperaturen liegen zwischen 50 und 300°C, vorzugsweise bei 50 bis 150°C, wenn in Lösung gearbeitet wird.

Die neuen Pyrazolopyrimidin-7-ylamine werden gegebenenfalls nach Verdampfen des Lösungsmittels oder Verdünnen mit Wasser als kristalline Verbindungen isoliert.

Die für die Herstellung der Pyrazolopyrimidin-7-ylamine benötigten substituierten Alkylcyanide der Formel VI sind teilweise bekannt oder können nach bekannten Methoden aus Alkylcyaniden und Carbonsäureestern mit starken Basen, z.B. Alkalihydriden, Alkalimetallalkoholaten, Alkaliamiden oder Metallalkylen, hergestellt werden [vgl.: J. Amer. Chem. Soc. Bd. 73, (1951) S. 3766].

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder ein- oder zweifach verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: Alkylgruppe wie voranstehend genannt, in der teilweise oder vollständig die Wasserstoffatome durch Halogenatome wie vorstehend genannt ersetzt sein können: insbesondere Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl;
Cycloalkyl: mono- oder bicyclische, gesättigte Kohlenwasserstoffgruppen mit 3 bis 6 Kohlenstoffringgliedern, wie Cyclopropyl, Cyclobutyl, Cyclopentyl und Cyclohexyl;
Alkoxyalkyl: gesättigte, geradkettige oder ein-, zwei- oder dreifach verzweigte Kohlenwasserstoffkette, die durch ein Sauerstoffatom unterbrochen ist, z. B. C₅-C₁₂-Alkoxyalkyl: Kohlenwasserstoffkette wie voranstehend beschreiben mit 5 bis 12 Kohlenstoffatomen, die durch ein Sauerstoffatom an beliebiger Stelle unterbrochen sein kann, wie Propoxy-ethyl, Butoxy-ethyl, Pentoxy-ethyl, Hexyloxy-ethyl, Heptyloxy-ethyl, Octyloxyethyl, Nonyloxy-ethyl, 3-(3-Ethyl-hexyloxy)-ethyl, 3-(2,4,4-Trimethyl-pentyloxy)-ethyl, 3-(1-Ethyl-3-methyl-butoxy)-ethyl, Ethoxy-propyl, Propoxy-propyl, Butoxy-propyl, Pentoxy-propyl, Hexyloxy-propyl, Heptyloxy-propyl, Octyloxy-propyl, Nonyloxy-propyl, 3-(3-Ethyl-hexyloxy)-propyl, 3-(2,4,4-Trimethyl-pentyloxy)-propyl, 3-(1-Ethyl-3-methyl-butoxy)-propyl, Ethoxy-butyl, Propoxy-butyl, Butoxy-butyl, Pentoxy-butyl, Hexyloxybutyl, Heptyloxy-butyl, Octyloxy-butyl, Nonyloxy-butyl, 3-(3-Ethyl-hexyloxy)-butyl, 3-(2,4,4-Trimethyl-pentyloxy)-butyl, 3-(1-Ethyl-3-methyl-butoxy)-butyl, Methoxy-pentyl, Ethoxy-pentyl, Propoxy-pentyl, Butoxy-pentyl, Pentoxy-pentyl, Hexyloxy-pentyl, Heptyloxy-pentyl, 3-(3-Methyl-hexyloxy)-pentyl, 3-(2,4-Dimethyl-pentyloxy)-pentyl, 3-(1-methyl-3-methyl-butoxy)-pentyl;
Alkenyl: ungesättigte, geradkettige oder verzweigte Kohlenwasserstoffreste mit 2 bis 4, 6, 8 oder 10 Kohlenstoffatomen und einer oder zwei Doppelbindungen in beliebiger Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Kohlenwasserstoffgruppen mit 2 bis 4 oder 6 Kohlenstoffatomen und einer oder zwei Dreifachbindungen in beliebiger Position, C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Alkylen: divalente unverzweigte Ketten, bevorzugt aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
Oxyalkylen: divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
Oxyalkylenoxy: divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;
In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der Pyrazolopyrimidinylamine der Formel I sind die folgenden Bedeutungen der Substituenten, und zwar jeweils für sich allein oder in Kombination, besonders bevorzugt:

Verbindungen I sind bevorzugt ,in denen die 6-Phenylgrupe durch eine bis drei Halogen- oder CH₂-C₁-C₄-Alkylgruppen substituiert ist.

Eine bevorzugte Ausgestaltung der Verbindungen der Formel I sind solche, in denen keine Gruppe R^{a} vorliegt.

Eine weitere bevorzugte Ausgestaltung betrifft der Verbindungen der Formel I, in der L¹ und L³ Wasserstoff bedeuten.

Eine weitere bevorzugte Ausgestaltung betrifft der Verbindungen der Formel I, in der L² und L³ Wasserstoff bedeuten.

Eine weitere bevorzugte Ausgestaltung betrifft der Verbindungen der Formel I, in der L¹ und L² ungleich Wasserstoff sind und L³ Wasserstoff bedeutet. Besonders bevorzugt sind solche Verbindungen, in denen L¹ und L² Halogen sind.

Eine weitere bevorzugte Ausgestaltung betrifft der Verbindungen der Formel I, in der eine Gruppe aus L¹, L² und L³ für Alkyl, insbesondere verzweigtes Alkyl, wie tert. Butyl steht.

Eine weitere bevorzugte Ausgestaltung betrifft der Verbindungen der Formel I, in der die 6-Phenylgruppe durch eine bis drei Gruppen Halogen, Cyano, Hydroxy, Mercapto, Nitro, NR^{A}R^{B}, C₁-C₁₀-Alkyl, C₁-C₆-Halogenalkyl, C₂-C₆-Alkenyl, C₂-C₆-Alkinyl und C₁-C₆-Alkoxy substituiert ist. Besonders bevorzugt trägt die Phenylgruppe zwei, insbesondere einen Substituenten.

Eine Ausgestaltung betrifft der Verbindungen der Formel I, in der R¹ Halogenmethyl, insbesondere Trifluormethyl bedeutet.

Eine weitere Ausgestaltung betrifft der Verbindungen der Formel I, in der R¹ Alkenyl, insbesondere Allyl bedeutet.

Eine weitere Ausgestaltung betrifft der Verbindungen der Formel I, in der R¹ Alkoxyalkyl, bevorzugt C₁-C₇-Alkoxymethyl, insbesondere Methoxymethyl bedeutet.

Eine weitere bevorzugte Ausgestaltung betrifft der Verbindungen der Formel I, in der R² nicht Wasserstoff ist.

In einer weiteren Ausführung der Verbindungen I stellt R² NH₂ oder C₁-C₄-Alkyl dar, bevorzugt C₁-C₂-Alkyl oder NH₂, insbesondere Methyl.

Insbesondere bevorzugt sind Verbindungen der Formel I, in denen L¹ für Cyano, Hydroxy, Mercapto, Nitro, NR^{A}R^{B}, C₁-C₆-Alkyl, Halogenmethyl, und C₁-C₂-Alkoxy steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I, in denen R¹ Trifluormethyl, R² Methyl bedeutet und die Kombination von L¹, L² und L³ für eine Verbindung jeweils einer Zeile der Tabelle A entspricht

### Tabelle 2

Verbindungen der Formel I, in denen R¹ Trifluormethyl, R² Amino bedeutet und die Kombination von L¹, L² und L³ für eine Verbindung jeweils einer Zeile der Tabelle A entspricht

### Tabelle 3

Verbindungen der Formel I, in denen R¹ Allyl, R² Methyl bedeutet und die Kombination von L¹, L² und L³ für eine Verbindung jeweils einer Zeile der Tabelle A entspricht

### Tabelle 4

Verbindungen der Formel I, in denen R¹ Allyl, R² Amino bedeutet und die Kombination von L¹, L² und L³ für eine Verbindung jeweils einer Zeile der Tabelle A entspricht

### Tabelle 5

Verbindungen der Formel I, in denen R¹ Methoxymethyl, R² Methyl bedeutet und die Kombination von L¹, L² und L³ für eine Verbindung jeweils einer Zeile der Tabelle A entspricht

### Tabelle 6

Verbindungen der Formel I, in denen R¹ Methoxymethyl, R² Amino bedeutet und die Kombination von L¹, L² und L³ für eine Verbindung jeweils einer Zeile der Tabelle A entspricht

**Tabelle A**

| Nr. | L¹ | L² | L³ |
|---|---|---|---|
| A-1 | CH₃ | H | H |
| A-2 | H | CH₃ | H |
| A-3 | CH₃ | CH₃ | H |
| A-4 | CH₃ | H | CH₃ |
| A-5 | CH₂CH₃ | H | H |
| A-6 | H | CH₂CH₃ | H |
| A-7 | CH₂CH₃ | CH₂CH₃ | H |
| A-8 | CH₂CH₃ | H | CH₂CH₃ |
| A-9 | CH₂CH₂CH₃ | H | H |
| A-10 | H | CH₂CH₂CH₃ | H |
| A-11 | CH₂CH₂CH₃ | CH₂CH₂CH₃ | H |
| A-12 | CH₂CH₂CH₃ | H | CH₂CH₂CH₃ |
| A-13 | CH(CH₃)₂ | H | H |
| A-14 | H | CH(CH₃)₂ | H |
| A-15 | CH(CH₃)₂ | H | CH(CH₃)₂ |
| A-16 | CH₂CH₂CH₂CH₃ | H | H |
| A-17 | H | CH₂CH₂CH₂CH₃ | H |
| A-18 | CH₂CH₂CH₂CH₃ | CH₂CH₂CH₂CH₃ | H |
| A-19 | CH₂CH₂CH₂CH₃ | H | CH₂CH₂CH₂CH₃ |
| A-20 | CH(CH₃)CH₂CH₃ | H | H |
| A-21 | H | CH(CH₃)CH₂CH₃ | H |
| A-22 | CH(CH₃)CH₂CH₃ | H | CH(CH₃)CH₂CH₃ |
| A-23 | C(CH₃)₃ | H | H |
| A-24 | H | C(CH₃)₃ | H |
| A-25 | C(CH₃)₃ | H | C(CH₃)₃ |
| A-26 | CH₂CH(CH₃)CH₂CH₃ | H | H |
| A-27 | H | CH₂CH(CH₃)CH₂CH₃ | H |
| A-28 | CH₂CH(CH₃)CH₂CH₃ | H | CH₂CH(CH₃)CH₂CH₃ |
| A-29 | CH₂CH(CH₂CH₃)₂ | H | H |
| A-30 | H | CH₂CH(CH₂CH₃)₂ | H |
| A-31 | CH₂CH(CH₂CH₃)₂ | H | CH₂CH(CH₂CH₃)₂ |
| A-32 | CH₂C(CH₃)₃ | H | H |
| A-33 | H | CH₂C(CH₃)₃ | H |
| A-34 | CH₂C(CH₃)₃ | H | CH₂C(CH₃)₃ |
| A-35 | CH₂C(CH₃)₂CH₂CH₃ | H | H |
| A-36 | H | CH₂C(CH₃)₂CH₂CH₃ | H |
| A-37 | CH₂C(CH₃)₂CH₂CH₃ | H | CH₂C(CH₃)₂CH₂CH₃ |
| A-38 | CH₂C(CH₂CH₃)₂CH₃ | H | H |
| A-39 | H | CH₂C(CH₂CH₃)₂CH₃ | H |
| A-40 | CH₂C(CH₂CH₃)₂CH₃ | H | CH₂C(CH₂CH₃)₂CH₃ |
| A-41 | Cl | H | H |
| A-42 | H | Cl | H |
| A-43 | Cl | Cl | H |
| A-44 | Cl | H | Cl |
| A-45 | Cl | Cl | Cl |
| A-46 | F | H | H |
| A-47 | H | F | H |
| A-48 | F | F | H |
| A-49 | F | H | F |
| A-50 | F | F | F |
| A-51 | Br | H | H |
| A-52 | H | Br | H |
| A-53 | Br | Br | H |
| A-54 | Br | H | Br |
| A-55 | Br | Br | Br |
| A-56 | CHF₂ | H | H |
| A-57 | H | CHF₂ | H |
| A-58 | CHF₂ | H | CHF₂ |
| A-59 | CHF₂ | CHF₂ | H |
| A-60 | CF₃ | H | H |
| A-61 | H | CF₃ | H |
| A-62 | CF₃ | H | CF₃ |
| A-63 | CF₃ | CF₃ | H |
| A-64 | CH=CH₂ | H | H |
| A-65 | H | CH=CH₂ | H |
| A-66 | CH=CH₂ | CH=CH₂ | H |
| A-67 | CH=CH₂ | H | CH=CH₂ |
| A-68 | CH₂CH=CH₂ | H | H |
| A-69 | H | CH₂CH=CH₂ | H |
| A-70 | CH₂CH=CH₂ | CH₂CH=CH₂ | H |
| A-71 | CH₂CH=CH₂ | H | CH₂CH=CH₂ |
| A-72 | C≡CH | H | H |
| A-73 | H | C≡CH | H |
| A-74 | C≡CH | H | C≡CH |
| A-75 | CH₂C≡CH | H | H |
| A-76 | H | CH₂C≡CH | H |
| A-77 | CH₂C≡CH | H | CH₂C≡CH |
| A-78 | OCH₃ | H | H |
| A-79 | H | OCH₃ | H |
| A-80 | OCH₃ | H | OCH₃ |
| A-81 | OCH₂CH₃ | H | H |
| A-82 | H | OCH₂CH₃ | H |
| A-83 | OCH₂CH₃ | H | OCH₂CH₃ |
| A-84 | OCH₂CH₂CH₃ | H | H |
| A-85 | H | OCH₂CH₂CH₃ | H |
| A-86 | OCH₂CH₂CH₃ | H | OCH₂CH₂CH₃ |
| A-87 | O(CH₂)₃CH₃ | H | H |
| A-88 | H | O(CH₂)₃CH₃ | H |
| A-89 | O(CH₂)₃CH₃ | H | O(CH₂)₃CH₃ |
| A-90 | O(CH₂)₄CH₃ | H | H |
| A-91 | H | O(CH₂)₄CH₃ | H |
| A-92 | O(CH₂)₄CH₃ | H | O(CH₂)₄CH₃ |
| A-93 | O(CH₂)₅CH₃ | H | H |
| A-94 | H | O(CH₂)₅CH₃ | H |
| A-95 | O(CH₂)₅CH₃ | H | O(CH₂)₅CH₃ |
| A-96 | OCH(CH₃)CH₂CH₃ | H | H |
| A-97 | H | OCH(CH₃)CH₂CH₃ | H |
| A-98 | OCH(CH₃)CH₂CH₃ | H | OCH(CH₃)CH₂CH₃ |
| A-99 | OCH₂CH(CH₃)CH₂CH₃ | H | H |
| A-100 | H | OCH₂CH(CH₃)CH₂CH₃ | H |
| A-101 | OCH₂CH(CH₃)CH₂CH₃ | H | OCH₂CH(CH₃)CH₂CH₃ |
| A-102 | OCH₂CH(CH₂CH₃)₂ | H | H |
| A-103 | H | OCH₂CH(CH₂CH₃)₂ | H |
| A-104 | OCH₂CH(CH₂CH₃)₂ | H | OCH₂CH(CH₂CH₃)₂ |
| A-105 | OC(CH₃)₃ | H | H |
| A-106 | H | OC(CH₃)₃ | H |
| A-107 | OC(CH₃)₃ | H | OC(CH₃)₃ |
| A-108 | OCH₂C(CH₃)₃ | H | H |
| A-109 | H | OCH₂C(CH₃)₃ | H |
| A-110 | OCH₂C(CH₃)₃ | H | OCH₂C(CH₃)₃ |
| A-111 | C₆H₅ | H | H |
| A-112 | H | C₆H₅ | H |
| A-113 | OC₆H₅ | H | H |
| A-114 | H | OC₆H₅ | H |
| A-115 | SC₆H₅ | H | H |
| A-116 | H | SC₆H₅ | H |
| A-117 | CH₂C₆H₅ | H | H |
| A-118 | H | CH₂C₆H₅ | H |
| A-119 | OCH₂C₆H₅ | H | H |
| A-120 | H | OCH₂C₆H₅ | H |
| A-121 | SCH₂C₆H₅ | H | H |
| A-122 | H | SCH₂C₆H₅ | H |

Die Verbindungen I eignen sich als Fungizide. Sie zeichnen sich aus durch eine hervorragende Wirksamkeit gegen ein breites Spektrum von pflanzenpathogenen Pilzen aus der Klasse der Ascomyceten, Deuteromyceten, Basidiomyceten und Peronosporomyceten (syn. Oomyceten). Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- Altemaria Arten an Gemüse, Raps, Zuckerrüben und Obst und Reis , wie z.B. A.solani oder A. alternata an Kartoffeln und Tomaten,
- Aphanomyces Arten an Zuckerrüben und Gemüse,
- Ascochyta-Arten an Getreide and Gemüse,
- Bipolaris- und Drechslera Arten an Mais, Getreide, Reis und Rasen, wie z.B. D.maydis an Mais,
- Blumeria graminis (Echter Mehltau) an Getreide,
- Botrytis cinerea (Grauschimmel) an Erdbeeren, Gemüse, Blumen und Weinreben,
- Bremia lactucae an Salat,
- Cercospora Arten an Mais, Sojabohnen, Reis und Zuckerrüben,
- Cochliobolus Arten an Mais, Getreide, Reis, wie z.B. Cochliobolus sativus an Getreide, Cochliobolus miyabeanus an Reis,
- Colletotricum Arten an Sojabohnen und Baumwolle,
- Drechslera Arten, Pyrenophora Arten an Mais, Getreide, Reis und Rasen, wie z.B. D.teres an Gerste oder D. tritici-repentis an Weizen,
- Esca an Weinrebe, verursacht durch Phaeoacremonium chlamydosporium, Ph. Aleophilum, und Formitipora punctata (syn. Phellinus punctatus),
- Exserohilum Arten an Mais,
- Erysiphe cichoracearum und Sphaerotheca fuliginea an Gurkengewächsen,
- Fusarium und Verticillium Arten an verschiedenen Pflanzen wie z.B. F. graminearum oder F. culmorum an Getreide oder F. oxysporum an einer Vielzahl von Pflanzen wie z.B. Tomaten,
- Gaeumanomyces graminis an Getreide,
- Gibberella arten an Getreide und Reis (z.B.. Gibberella fujikuroi an Reis),
- Grainstaining complex an Reis,
- Helminthosporium Arten an Mais und Reis,
- Michrodochium nivale an Getreide,
- Mycosphaerella Arten an Getreide, Bananen und Erdnüssen, wie z.B. M. graminicola an Weizen oder M.fijiensis an Bananen,
- Peronospora-Arten an Kohl und Zwiebelgewächsen, wie z.B. P. brassicae an Kohl oder P. destructor an Zwiebel,
- Phakopsara pachyrhizi und Phakopsara meibomiae an Sojabohnen,
- Phomopsis Arten an Sojabohnen und Sonnenblumen,
- Phytophthora infestans an Kartoffeln und Tomaten,
- Phytophthora Arten an verschiedenen Pflanzen wie z.B. P.capsici an Paprika,
- Plasmopara viticola an Weinreben,
- Podosphaera leucotricha an Apfel,
- Pseudocercosporella herpotrichoides an Getreide,
- Pseudoperonospora an verschiedenen Pflanzen wie z.B. P. cubensis an Gurke oder P. humili an Hopfen,
- Puccinia Arten an verschiedenen Pflanzen wie z.B. P. triticina, P. striformins, P. hordei oder P.graminis an Getreide, oder P. asparagi an Spargel,
- Pyricularia oryzae, Corticium sasakii, Sarocladium oryzae, S.attenuatum, Entyloma oryzae, an Reis,
- Pyricularia grisea an Rasen und Getreide,
- Pythium spp. an Rasen, Reis, Mais, Baumwolle, Raps, Sonnenblumen, Zuckerrüben, Gemüse und anderen Pflanzen wie z.B. P.ultiumum an verschiedenen Pflanzen, P. aphanidermatum an Rasen,
- Rhizoctonia-Arten an Baumwolle, Reis, Kartoffeln, Rasen, Mais, Raps, Zuckerrüben, Gemüse und an verschiedenen Pflanzen wie z.B. R.solani an Rüben und verschiedenen Pflanzen,
- Rhynchosporium secalis an Gerste, Roggen und Triticale,
- Sclerotinia Arten an Raps und Sonnenblumen,
- Septoria tritici und Stagonospora nodorum an Weizen,
- Erysiphe (syn. Uncinula) necator an Weinrebe,
- Setospaeria Arten an Mais und Rasen,
- Sphacelotheca reilinia an Mais,
- Thievaliopsis Arten an Sojabohnen und Baumwolle,
- Tilletia Arten an Getreide,
- Ustilago-Arten an Getreide, Mais und Zuckerrohr, wie z.B. U. maydis an Mais,
- Venturia-Arten (scharf) an Äpfeln und Birnen wie. z.B. V. inaequalis an Apfel.

Insbesondere eignen sie sich zur Bekämpfung von Schadpilzen aus der Klasse der Peronosporomycetes (syn.Oomyceten), wie Peronospora-Arten, Phytophthora-Arten, Plasmopara viticola und Pseudoperonospora-Arten.

Die Verbindungen I eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie Ophiostoma spp., Ceratocystis spp., Aureobasidium pullulans, Sclerophoma spp., Chaetomium spp., Humicola spp., Petriella spp., Trichurus spp.; Basidiomyceten wie Coniophora spp., Coriolus spp., Gloeophyllum spp., Lentinus spp., Pleurotus spp., Poria spp., Serpula spp. und Tyromyces spp., Deuteromyceten wie Aspergillus spp., Cladosporium spp., Penicillium spp., Trichoderma spp., Alternaria spp., Paecilomyces spp. und Zygomyceten wie Mucor spp., darüber hinaus im Materialschutz folgende Hefepilze: Candida spp. und Saccharomyces cerevisae.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung, z.B. durch Bestäuben, Beschichten oder Tränken von Saatgut, werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Formulierungen für die Saatgutbehandlung können zusätzlich Bindemittel und/oder Geliermittel und gegebenenfalls Farbstoffe enthalten.

Bindemittel können zugesetzt werden, um Haftung der Wirkstoffe auf dem Saatgut nach der Behandlung zu erhöhen. Geeignete Bindemittel sind beispielsweise EO/PO Blockcopolymer-Tenside, aber auch Polyvinylalcohole, Ppolyvinylpyrrolidone, Polyacrylate, Polymethacrylate, Polybutene, Polyisobutylene, Polystyrole, Polyethylenamine, Polyethylenamide, Polyethylenimine (Lupasol®, Polymin®), Polyether, Polyurethane, Polyvinylacetate, Tylose und Copolymere aus diesen Polymeren. Ein geeignetes Geliermittel ist beispielsweise Carrageen (Satiagel®).

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Für die Saatgutbehandlung ergeben die betreffenden Formulierungen nach zwei- bis zehnfacher Verdünnung Wirkstoffkonzentrationen von 0,01 bis 60 Gew.-%, bevorzugt 0,1 bis 40 Gew.-% in den fertig verwendbaren Zubereitungen.

Beispiele für erfindungsgemäße Formulierungen sind:
1. Produkte zur Verdünnung in Wasser
   A Wasserlösliche Konzentrate (SL, LS)
      10 Gew.-Teile einer erfindungsgemäßen Verbindung werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.
   B Dispergierbare Konzentrate (DC)
      20 Gew.-Teile einer erfindungsgemäßen Verbindung werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%
   C Emulgierbare Konzentrate (EC)
      15 Gew.-Teile einer erfindungsgemäßen Verbindung werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.
   D Emulsionen (EW, EO, ES)
      25 Gew.-Teile einer erfindungsgemäßen Verbindung werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.
   E Suspensionen (SC, OD, FS) .
      20 Gew.-Teile einer erfindungsgemäßen Verbindung werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-% .
   F Wasserdispergierbare und wasserlösliche Granulate (WG, SG)
      50 Gew.-Teile einer erfindungsgemäßen Verbindung werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.
   G Wasserdispergierbare und wasserlösliche Pulver (WP, SP, SS, WS)
      75 Gew.-Teile einer erfindungsgemäßen Verbindung werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.
   H Gelformulierungen
      In einer Kugelmühle werden 20 Gew.-Teile einer erfindungsgemäßen Verbindung, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.
2. Produkte für die Direktapplikation
   I Stäube (DP, DS)
      5 Gew.-Teile einer erfindungsgemäßen Verbindung werden fein gemahlen und mit 95 Gew.-Teilen feinteiligem Kaolin innig vermischt. Man erhält dadurch ein Stäubemittel mit 5 Gew.-% Wirkstoffgehalt.
   J Granulate (GR, FG, GG, MG)
      0,5 Gew-Teile einer erfindungsgemäßen Verbindung werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.
   K ULV- Lösungen (UL)
      10 Gew.-Teile einer erfindungsgemäßen Verbindung werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Bevorzugt werden FS Formulierungen für die Saatgutbehandlung verwendet. Üblicherweise enthalten solche Formulierungen 1 bis 800 g/l Wirkstoff, 1 bis 200 g/l Tenside, 0 bis 200 g/l Frostschutzmittel, 0 bis 400 g/l Bindemittel, 0 bis 200 g/l Farbstoffe und Lösungsmittel, vorzugsweise Wasser.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:100 bis 100:1, bevorzugt 1:10 bis 10:1 zugemischt werden.

Als Adjuvants in diesem Sinne kommen insbesondere in Frage: organisch modifizierte Polysiloxane, z.B. Break Thru S 240®; Alkoholalkoxylate, z. B. Atplus 245®, Atplus MBA 1303®, Plurafac LF 300® und Lutensol ON 30®; EO-PO-Blockpolymerisate, z. B. Pluronic RPE 2035® und Genapol B®; Alkoholethoxylate, z. B. Lutensol XP 80®; und Natriumdioctylsulfosuccinat, z. B. Leophen RA®.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen (I) bzw. der sie enthaltenden Mittel mit einem oder mehreren weiteren Wirkstoffen, insbesondere Fungiziden, kann in vielen Fällen das Wirkungsspektrum verbreitert oder Resistenzentwicklungen vorgebeugt werden. In vielen Fällen erhält man dabei synergistische Effekte.

Die folgende Liste von Fungiziden, mit denen die erfindungsgemäßen Verbindungen gemeinsam angewendet werden können, soll die Kombinationsmöglichkeiten erläutern, nicht aber einschränken:
Strobilurine
   Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-(2,5-Dimethylphenyl-oxymethylen)phenyl)-3-methoxy-acrylsäuremethylester;
Carbonsäureamide
   - Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3-Difluormethyl-1-methyl-pyrazol-4-carbonsäure-(3',4'-dichlor-5-fluor-biphenyl-2-yl)-amid, 3,4-Dichlor-isothiazol-5-carbonsäure-(2-cyano-phenyl)-amid;
   - Carbonsäuremorpholide: Dimethomorph, Flumorph;
   - Benzoesäureamide: Flumetover, Fluopicolide (Picobenzamid), Zoxamide;
   - Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;
Azole
   - Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazol, Myclobutanil, Penconazole, Propiconazole, Prothioconazole, Simeconazole, Tebuconazole, Tetraconazole, Triadimenol, Triadimefon, Triticonazole;
   - Imidazole: Cyazofamid, Imazalil, Pefurazoate, Prochloraz, Triflumizole;
   - Benzimidazole: Benomyl, Carbendazim, Fuberidazole, Thiabendazole;
   - Sonstige: Ethaboxam, Etridiazole, Hymexazole;
Stickstoffhaltige Heterocyclylverbindungen
   - Pyridine: Fluazinam, Pyrifenox, 3-[5-(4-Chlor-phenyl)-2,3-dimethyl-isoxazolidin-3-yl]-pyridin;
   - Pyrimidine: Bupirimate, Cyprodinil, Ferimzone, Fenarimol, Mepanipyrim, Nuarimol, Pyrimethanil;
   - Piperazine: Triforine;
   - Pyrrole: Fludioxonil, Fenpiclonil;
   - Morpholine: Aldimorph, Dodemorph, Fenpropimorph, Tridemorph;
   - Dicarboximide: Iprodione, Procymidone, Vinclozolin;
   - sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Pyroquilon, Quinoxyfen, Tricyclazole, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluor-2-methyl-indol-1-sulfonyl)-[1,2,4]triazol-1-sulfonsäuredimethylamid;
Carbamate und Dithiocarbamate
   - Dithiocarbamate: Ferbam, Mancozeb, Maneb, Metiram, Metam, Propineb, Thiram, Zineb, Ziram;
   - Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester;
Sonstige Fungizide
   - Guanidine: Dodine, Iminoctadine, Guazatine;
   - Antibiotika: Kasugamycin, Polyoxine, Streptomycin, Validamycin A;
   - Organometallverbindungen: Fentin Salze;
   - Schwefelhaltige Heterocyclylverbindungen: Isoprothiolane, Dithianon;
   - Organophosphorverbindungen: Edifenphos, Fosetyl, Fosetyl-aluminium, Iprobenfos, Pyrazophos, Tolclofos-methyl, Phosphorige Säure und ihre Salze;
   - Organochlorverbindungen: Thiophanate Methyl, Chlorothalonil, Dichlofluanid, Tolylfluanid, Flusulfamide, Phthalide, Hexachlorbenzene, Pencycuron, Quintozene;
   - Nitrophenylderivate: Binapacryl, Dinocap, Dinobuton;
   - Anorganische Wirkstoffe: Bordeaux Brühe, Kupferacetat, Kupferhydroxid, Kupferoxychlorid, basisches Kupfersulfat, Schwefel;
   - Sonstige: Spiroxamine, Cyflufenamid, Cymoxanil, Metrafenone.

### Synthesebeispiele

Die in dem nachstehenden Synthesebeispiel wiedergegebene Vorschrift wurde unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Synthese von 2-Methyl-5-Trifluormethyl-6-(p-t-butylphenyl)-7-aminopyrazolopyrimidin

Eine Suspension von 0,5g (1,8 mmol) 1-(4-t-butylphenyl)-3-Trifluor-2-oxo-propan-1-nitril, 0,18 g (1,8mmol) 3-Amino-5-methyl-1,2-pyrazol und 0,07 mg (0,36mmol) p-Toluolsulfonsäure in 5 ml Mesitylen wurde 5 Std. auf 160°C am Wasserabscheider erhitzt. Danach wurde das Mesitylen abdestilliert und der Rückstand aus Dichlormethan / Wasser digeriert. Nach Trocknung und Abdestillieren des Lösungsmittels blieben 0,11 g der Titelverbindung in Form farbloser Kristalle zurück.

| Nr. | R² | L¹ | L² | L³ | R¹ | phys. Daten (Fp. [°C]; ¹H-NMR δ [ppm]) |
|---|---|---|---|---|---|---|
| I-1 | CH₃ | H | C(CH₃)₃ | H | CF₃ | 242-243 |
| I-2 | CH₃ | H | C(CH₃)₃ | H | CH₂[4-C(CH₃)₃C₆H₄] | 214-215 |
| I-3 | CH₃ | H | F | H | CF₃ | 2,4 (s); 6,4 (s); 7,2-7,4 (m) |
| I-4 | CH₃ | H | F | H | CH₂OCH₃ | 2,4 (s); 3,1 (s); 4,0 (s); 6,2 (s); 6,9 (s); 7,2-7,4 (m) |
| I-5 | CH₃ | H | Cl | H | CF₃ | 2,4 (s); 6,4 (s); 7,3-7,6 (m) |
| I-6 | H | H | F | H | CF₃ | 6,7 (s); 7,2 (m); 7,4 (m); 7,5 (s); 8,3 (s) |
| I-7 | H | H | Cl | H | CF₃ | 6,7 (s); 7,3 (m); 7,5-7,7 (m); 8,3 (s) |
| I-8 | CH₃ | F | H | H | CF₃ | 2,4 (s); 6,4 (s); 6,7 (s); 7,1-7,5 (m) |
| I-9 | CH₃ | F | H | H | CH₂C₆H₅ | 2,35 (s); 3,7 (s); 6,1 (s); 6,8 (s); 6,9-7,5 (m) |
| I-10 | CH₃ | H | Cl | H | CH₂C₆H₅ | 2,35 (s); 3,7 (s); 6,1 (s); 6,7 (s); 6,9 (d); 7,0-7,2 (m); 7,4 (d) |
| I-11 | CH₃ | H | F | H | CH₂C₆H₅ | 2,4 (s); 3,7 (s); 6,15 (s); 6,8 (s); 6,9 (d); 7,0-7,3 (m) |
| I-12 | CH₃ | Cl | Cl | H | CH₂OCH₃ | 2,4 (s); 3,1 (s); 4,1 (s); 6,2 (s); 7,1 (s); 7,3 (d); 7,5, (s); 7,7 (d) |

### Anwendungsbeispiele

Die fungizide Wirkung der erfindungsgemäßen Verbindungen ließ sich durch folgende Versuche zeigen:

Die Wirkstoffe wurden als eine Stammlösung aufbereitet mit 25 mg Wirkstoff, welcher mit einem Gemisch aus Aceton und/oder DMSO und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegebenen Wirkstoffkonzentration verdünnt.

Anwendungsbeispiel - Wirksamkeit gegen Weizenmehltau verursacht durch Erysiphe [syn. Blumeria] graminis forma specialis. tritici

Blätter von in Töpfen gewachsenen Weizenkeimlingen wurden mit wässriger Suspension in der unten angegebenen Wirkstoffkonzentration bis zur Tropfnässe besprüht. Die Suspension oder Emulsion wurde wie oben beschrieben hergestellt. 24 Stunden nach dem Antrocknen des Spritzbelages mit Sporen des Weizenmehltaus (Erysiphe [syn. Blumeria] graminis forma specialis. tritici) bestäubt. Die Versuchspflanzen wurden anschließend im Gewächshaus bei Temperaturen zwischen 20 und 24°C und 60 bis 90 % relativer Luftfeuchtigkeit ausgestellt. Nach 7 Tagen wurde das Ausmaß der Mehftauentwicklung visuell in % Befall der gesamten Blattfläche ermittelt.

In diesem Test zeigten die mit 250 ppm des Wirkstoffs I-3 nur 20% Befall, während die unbehandelten Pflanzen zu 90% befallen waren.

## Patentansprüche

1. 6-Phenyl-pyrazolopyrimidin-7-ylamine der Formel I 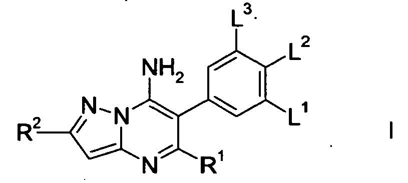 in der die Substituenten folgende Bedeutung haben:
L¹,L²,L³ unabhängig voneinander Wasserstoff, Halogen, Hydroxy, Mercapto, Nitro, NR^{A}R^{B}, C₁-C₁₀-Alkyl, C₁-C₄-Halogenalkyl, C₂-C₆-Alkenyl, C₂-C₆-Alkinyl, C₁-C₈-Alkoxy, Phenyl, Phenoxy, Phenylthio, Benzyloxy und Benzylthio;
R^{A}, R^{B} Wasserstoff und C₁-C₆-Alkyl;
wobei zwei benachbarte Gruppen aus L¹, L² und L³ gemeinsam eine C₁-C₄-Alkylen, C₂-C₄-Oxyalkylen, C₁-C₃-Oxyalkylenoxy oder Butadienylgruppe darstellen können;
wobei mindestens eine Gruppe L¹, L² oder L³ ungleich Wasserstoff ist und die Gruppen L¹, L² oder L³ unsubstituiert oder durch eine bis vier gleiche oder verschiedene Gruppen R^{a} substituiert sind:
R^{a} Halogen, Cyano, Hydroxy, Mercapto, C₁-C₁₀-Alkyl, C₁-C₁₀-Halogenalkyl, C₃-C₈-Cycloalkyl, C₂-C₁₀-Alkenyl, C₂-C₁₀-Alkinyl, C₁-C₆-Alkoxy, C₁-C₆-Alkylthio, C₁-C₆-Alkoxy-C₁-C₆-alkyl oder NR^{A}R^{B};
R¹ C₁-C₄-Halogenalkyl, C₂-C₆-Alkenyl, C₂-C₆-Alkinyl, C₂-C₈-Alkoxyalkyl oder Phenyl-C₁-C₂-alkyl, wobei der Ring unsubstituiert oder durch eine oder mehrere Gruppen Halogen oder C₁-C₈-Alkyl substituiert sein kann;
R² Wasserstoff, Halogen, Cyano, NR^{A}R^{B}, Hydroxy, Mercapto, C₁-C₆-Alkyl, C₁-C₆-Halogenalkyl, C₃-C₈-Cycloalkyl, C₁-C₆-Alkoxy, C₁-C₆-Alkylthio, C₃-C₈-Cycloalkoxy, C₃-C₈-Cycloalkylthio, Carboxyl, Formyl, C₁-C₁₀-Alkylcarbonyl, C₁-C₁₀-Alkoxycarbonyl, C₂-C₁₀-Alkenyloxycarbonyl, C₂-C₁₀-Alkinyloxycarbonyl, Phenyl, Phenoxy, Phenylthio, Benzyloxy, Benzylthio, C₁-C₆-Alkyl-S(O)ₘ- und fünf- oder sechsgliedriger gesättigter, partiell ungesättigter oder aromatischer Heterocyclus, enthaltend ein bis vier Heteroatome aus der Gruppe O, N oder S;
m 0, 1 oder 2;
wobei die cyclischen Gruppen in L¹, L², L³, R^{a} und/oder R¹ unsubstituiert oder durch eine bis vier Gruppen R^{b} substituiert sind:
R^{b} Halogen, Cyano, Hydroxy, Mercapto, Nitro, NR^{A}R^{B}, C₁-C₁₀-Alkyl, C₁-C₆-Halogenalkyl, C₂-C₆-Alkenyl, C₂-C₆-Alkinyl, C₁-C₆-Alkoxy und fünf- oder sechsgliedriger gesättigter, partiell ungesättigter oder aromatischer Heterocyclus, enthaltend ein bis vier Heteroatome aus der Gruppe O, N oder S, welcher unsubstituiert oder durch eine oder mehrere Gruppen Halogen und/oder C₁-C₄-Alkyl substituiert sein kann.

2. Verbindungen der Formel I gemäß Anspruch 1, in der R¹ CF₃ oder Benzyl, welches unsubstituiert oder im Phenylteil durch Halogen oder C₁-C₄-Alkyl substituiert ist, bedeutet.

3. Verbindungen der Formel I gemäß Anspruch 1 oder 2, worin R² Methyl oder Amino bedeutet.

4. Verbindungen der Formel I gemäß einem der Ansprüche 1 bis 3, worin L³ Wasserstoff bedetuet.

5. Verfahren zur Herstellung von Verbindungen der Formel I gemäß einem der Ansprüche 1 bis 4, **dadurch gekennzeichnet, dass** man β-Ketoester der Formel II, 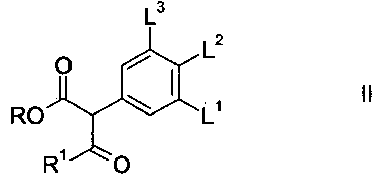 in der R für C₁-C₄-Alkyl steht, mit einem Aminopyrazol der Formel III 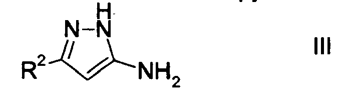 zu 7-Hydroxypyazolopyrimidinen der Formel IV 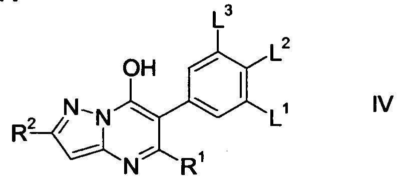 umsetzt, welche zu Verbindungen der Formel V, 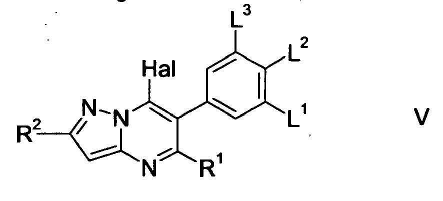 in der Hal für Chlor oder Brom steht, halogeniert werden, und V mit Ammoniak umgesetzt wird.

6. Verbindungen der Formel IV und V gemäß Anspruch 5.

7. Verfahren zur Herstellung von Verbindungen der Formel I gemäß Anspruch 1, **dadurch gekennzeichnet, dass** man Acylcyanide der Formel VI, 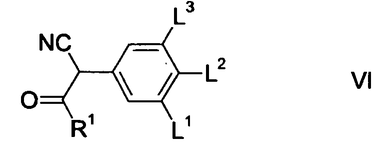 mit einem Aminopyrazol der Formel III gemäß Anspruch 5 umsetzt.

8. Fungizides Mittel, enthaltend einen festen oder flüssigen Träger und eine Verbindung der Formel I gemäß Anspruch 1.

9. Saatgut, enthaltend eine Verbindung der Formel I gemäß Anspruch 1 in einer Menge von 1 bis 1000 g pro 100 kg.

10. Verfahren zur Bekämpfung von pflanzenpathogenen Schadpilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß Anspruch 1 behandelt.

## Claims

1. A 6-phenylpyrazalopyrimidin-7-ylamine of the formula I 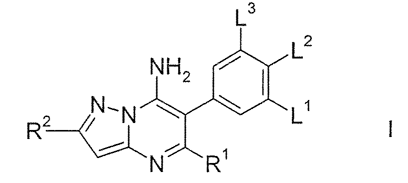 in which the substituents are as defined below:
L¹,L²,L³ independently of one another are hydrogen, halogen, hydroxyl, mercapto, nitro, NR^{A}R^{B}, C₁-C₁₀-alkyl, C₁-C₄-haloalkyl, C₂-C₆-alkenyl, C₂-C₆-alkynyl, C₁-C₈-alkoxy, phenyl, phenoxy, phenylthio, benzyloxy or benzylthio; R^{A}, R^{B} are hydrogen or C₁-C₆-alkyl;
where two adjacent groups from the group consisting of L¹, L² and L³ together may be a C₁-C₄-alkylene, C₂-C₄-oxyalkylene, C₁-C₃-oxyalkyleneoxy or butadienyl group;
where at least one group L¹, L² or L³ is not hydrogen and the groups L¹, L² or L³ are unsubstituted or substituted by one to four identical or different groups R^{a}:
R^{a} is halogen, cyano, hydroxyl, mercapto, C₁-C₁₀-alkyl, C₁-C₁₀-haloalkyl, C₃-C₈-cycloalkyl, C₂-C₁₀-alkenyl, C₂-C₁₀-alkynyl, C₁-C₆-alkoxy, C₁-C₆-alkylthio, C₁-C₆-alkoxy-C₁-C₆-alkyl or NR^{A}R^{B};
R¹ is C₁-C₄-haloalkyl, C₂-C₆-alkenyl, C₂-C₆-alkynyl, C₂-C₈-alkoxyalkyl or phenyl-C₁-C₂-alkyl, where the ring is unsubstituted or may be substituted by one or more halogen or C₁-C₈-alkyl groups;
R² is hydrogen, halogen, cyano, NR^{A}R^{B}, hydroxyl, mercapto, C₁-C₆-alkyl, C₁-C₆-haloalkyl, C₃-C₈-cycloalkyl, C₁-C₆-alkoxy, C₁-C₆-alkylthio, C₃-C₈-cycloalkoxy, C₃-C₈-cycloalkylthio, carboxyl, formyl, C₁-C₁₀-alkylcarbonyl, C₁₋-C₁₀-alkoxycarbonyl, C₂-C₁₀-alkenyloxycarbonyl, C₂-C₁₀-alkynyloxycarbonyl, phenyl, phenoxy, phenylthio, benzyloxy, benzylthio, C₁-C₆-alkyl-S(O)ₘ- or a five- or six-membered saturated, partially unsaturated or aromatic heterocycle which contains one to four heteroatoms from the group consisting of O, N and S;
m is 0, 1 or 2;
where the cyclic groups in L¹, L², L³, R^{a} and/or R¹ are unsubstituted or substituted by one to four groups R^{b}:
R^{b} is halogen, cyano, hydroxyl, mercapto, nitro, NR^{A}R^{B}, C₁-C₁₀-alkyl, C₁-C₆-haloalkyl, C₂-C₆-alkenyl, C₂-C₆-alkynyl, C₁-C₆-alkoxy or a five- or six-membered saturated, partially unsaturated or aromatic heterocycle which contains one to four heteroatoms from the group consisting of O, N or S which may be unsubstituted or substituted by one or more halogen and/or C₁-C₄-alkyl groups.

2. The compound of the formula I according to claim 1 in which R¹ is CF₃ or benzyl, which is unsubstituted or substituted in the phenyl moiety by halogen or C₁-C₄-alkyl.

3. The compound of the formula I according to claim 1 or 2 in which R² is methyl or amino.

4. The compound of the formula I according to any of claims 1 to 3 in which L³ is hydrogen.

5. A process for preparing compounds of the formula I according to any of claims 1 to 4 wherein β-keto esters of the formula II, 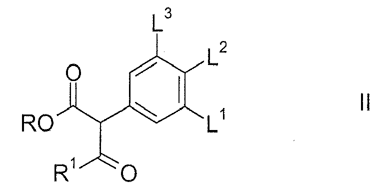 in which R is C₁-C₄-alkyl are reacted with an aminopyrazole of the formula III 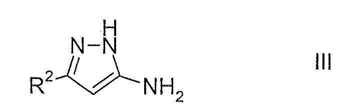 to give 7-hydroxypyrazolopyrimidines of the formula IV 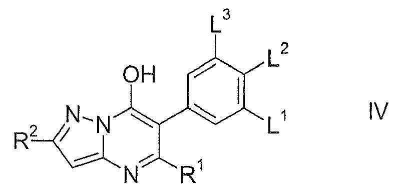 which are halogenated to give compounds of the formula V 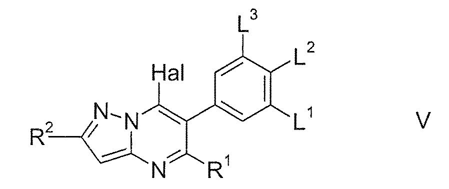 in which Hal is chlorine or bromine, and V is reacted with ammonia.

6. A compound of the formula IV or V according to claim 5.

7. A process for preparing compounds of the formula I according to claim 1 wherein acyl cyanides of the formula VI, 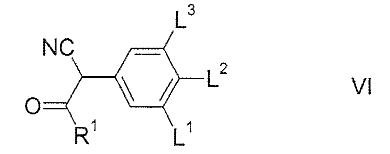 are reacted with an aminopyrazole of the formula III according to claim 5.

8. A fungicidal composition comprising a solid or liquid carrier and a compound of the formula I according to claim 1.

9. Seed comprising a compound of the formula I according to claim 1 in an amount of 1 to 1000 g per 100 kg.

10. A method for controlling phytopathogenic harmful fungi, wherein the fungi or the materials, plants, the soil or seed to be protected against fungal attack are treated with an effective amount of a compound of the formula I according to claim 1.

## Revendications

1. 6-phényl-pyrazolopyrimidin-7-ylamines de formule I 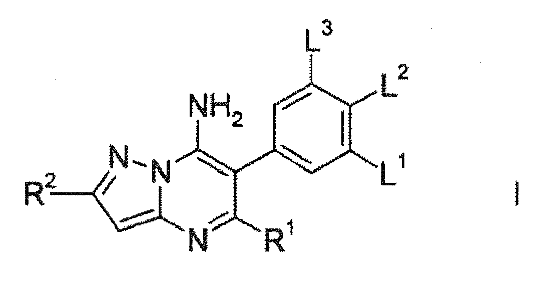 dans laquelle les substituants ont les significations suivantes :
L¹, L², L³ représentent, chacun indépendamment, un atome d'hydrogène ou d'halogène ou un groupe hydroxy, mercapto, nitro, NR^{A}R^{B}, alkyle en C₁-C₁₀, halogénoalkyle en C₁-C₄, alcényle en C₂-C₆, alcynyle en C₂-C₆, alcoxy en C₁-C₈, phényle, phénoxy, phénylthio, benzyloxy ou benzylthio ;
R^{A}, R^{B} représentent un atome d'hydrogène ou un groupe alkyle en C₁-C₆ ;
deux groupes contigus parmi L¹, L² et L³ pouvant former ensemble un groupe alkylène en C₁-C₄, oxyalkylène en C₂-C₄, oxyalkylène(C₁-C₃)oxy ou butadiényle ;
au moins un groupe L¹, L² ou L³ étant différent d'un atome d'hydrogène et les groupes L¹, L² et L³ étant non substitués ou substitués par un à quatre groupes R^{a} identiques ou différents :
R^{a} étant un atome d'halogène ou un groupe cyano, hydroxy, mercapto, alkyle en C₁-C₁₀, halogénoalkyle en C₁-C₁₀, cycloalkyle en C₂-C₈, alcényle en C₂-C₁₀, alcynyle en C₂-C₁₀, alcoxy en C₁-C₆, alkyl(C₁-C₆)thio, alcoxy(C₁-C₆)-alkyle-(C₁-C₆) ou NR^{A}R^{B} ;
R¹ représente un groupe halogénoalkyle en C₁-C₄, alcényle en C₂-C₆, alcynyle en C₂-C₆, alcoxyalkyle en C₂-C₈ ou phényl-alkyle(C₁-C₂), le cycle pouvant être non substitué ou substitué par un ou plusieurs groupes halogéno ou alkyle en C₁-C₈ ;
R² représente un atome d'hydrogène ou d'halogène ou un groupe cyano, NR^{A}R^{B}, hydroxy, mercapto, alkyle en C₁-C₆, halogénoalkyle en C₁-C₆, cycloalkyle en C₃-C₈, alcoxy en C₁-C₆, alkyl (C₁-C₆)thio, cycloalcoxy en C₃-C₈, cycloalkyl(C₃-C₈)thio, carboxy, formyle, alkyl(C₁-C₁₀)carbonyle, alcoxy(C₁-C₁₀)-carbonyle, alcényloxy(C₂-C₁₀) carbonyle, alcynyloxy(C₂-C₁₀)carbonyle, phényle, phénoxy, phénylthio, benzyloxy, benzylthio, alkyl(C₁-C₆)-S(O)ₘ-, ou un hétérocycle à cinq ou six chaînons, saturé, partiellement insaturé ou aromatique, contenant de un à quatre hétéroatomes choisis parmi O, N et S ;
m est 0, 1 ou 2 ;
les groupes cycliques dans L¹, L², L³, R^{a} et/ou R¹ étant non substitués ou substitués par un à quatre groupes R^{b} ;
R^{b} représente un atome d'halogène, un groupe cyano, hydroxy, mercapto, nitro, NR^{A}R^{B}, alkyle en C₁-C₁₀, halogénoalkyle en C₁-C₆, alcényle en C₂-C₆, alcynyle en C₂-C₆, alcoxy en C_{I}-C₆ ou un hétérocycle à cinq ou six chaînons, saturé, partiellement insaturé ou aromatique, contenant de un à quatre hétéroatomes choisis parmi O, N et S, qui peut être non substitué ou substitué par un ou plusieurs groupes halogéno et/ou alkyle en C₁-C₄.

2. Composés de formule I selon la revendication 1, dans lesquels R¹ représente CF₃ ou un groupe benzyle qui est non substitué ou substitué sur le fragment phényle par halogène ou alkyle en C₁-C₄.

3. Composés de formule I selon la revendication 1 ou 2, dans lesquels R² représente un groupe méthyle ou amino.

4. Composés de formule I selon l'une quelconque des revendications 1 à 3, dans lesquels L³ représente un atome d'hydrogène.

5. Procédé pour la préparation des composés de formule I selon l'une quelconque des revendications 1 à 4, **caractérisé en ce qu'**on fait réagir des β-cétoesters de formule II, 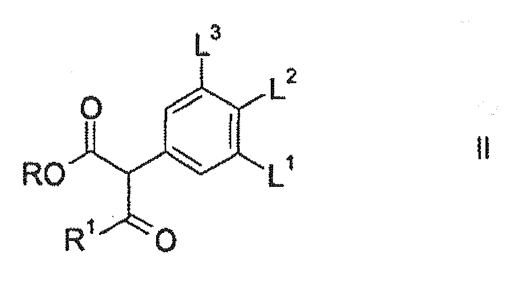 dans laquelle R représente un groupe alkyle en C₁-C₄, avec un aminopyrazole de formule III 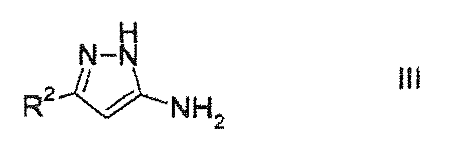 pour aboutir à des 7-hydroxypyrazolopyrimidines de formule IV 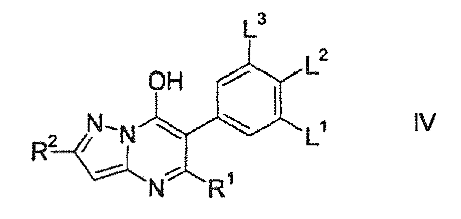 qui sont halogénées en composés de formule V, 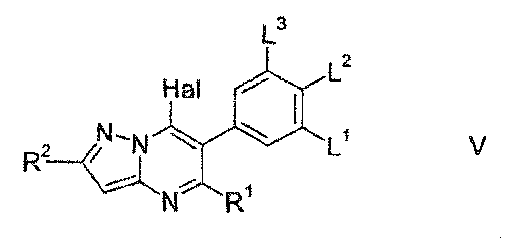 dans laquelle Hal représente le chlore ou le brome, et on fait réagir V avec de l'ammoniac.

6. Composés de formules IV et V selon la revendication 5.

7. Procédé pour la préparation de composés de formule I selon la revendication 1, **caractérisé en ce qu'**on fait réagir des acylcyanures de formule VI, 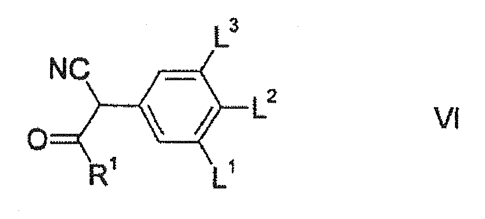 avec un aminopyrazole de formule III selon la revendication 5.

8. Composition fongicide, contenant une matière de support solide ou liquide et un composé de formule I selon la revendication 1.

9. Semence, contenant un composé de formule I selon la revendication 1, en une quantité de 1 à 1 000 g pour 100 kg.

10. Procédé pour la lutte contre des champignons nuisibles phytopathogènes, **caractérisé en ce qu'**on traite par une quantité efficace d'un composé de formule I selon la revendication 1 les champignons ou les matériaux, les plantes, le sol ou les semences, à protéger contre l'attaque de champignons.
